(19) Europäisches Patentamt
European Patent Office
Office européen des brevets

(11) EP 0 556 110 B1

(12) FASCICULE DE BREVET EUROPEEN

(45) Date de publication et mention de la délivrance du brevet: **02.05.1997 Bulletin 1997/18**

(51) Int. Cl.[6]: **A61K 9/51**, A61K 9/16

(21) Numéro de dépôt: **93400318.7**

(22) Date de dépôt: **09.02.1993**

# (54) Vecteur pour principe(s) actif(s) thérapeutique(s) ou cosmétique(s) à usage externe et composition thérapeutique ou cosmétique comprenant un tel vecteur

Vektor zur äusserlichen Anwendung von therapeutischen oder kosmetischen Wirkstoffen und diesen Vektor enthaltende therapeutische oder kosmetische Zusammensetzung

Vector for therapeutic or cosmetic drugs for external use and therapeutic or cosmetic composition containing such a vector

(84) Etats contractants désignés:
**BE CH DE DK ES FR GB GR IE IT LI LU MC NL PT SE**

(30) Priorité: **10.02.1992 FR 9201458**

(43) Date de publication de la demande:
**18.08.1993 Bulletin 1993/33**

(73) Titulaire: **EXSYMOL S.A.M.**
**MC-98000 Monte Carlo (MC)**

(72) Inventeurs:
- **Franco, André**
  **F-06500 Menton (FR)**
- **Gueyne, Jean**
  **MC-98000 Monaco (MC)**
- **Nicolay, Jean-François**
  **F-06230 Villefranche sur Mer (FR)**
- **Seguin, Marie-Christine**
  **MC-98000 Monaco (MC)**

(74) Mandataire: **Kedinger, Jean-Paul et al**
**c/o Cabinet Malemont**
**42, avenue du Président Wilson**
**75116 Paris (FR)**

(56) Documents cités:
**EP-A- 0 313 867**
**EP-A- 0 409 690**
**WO-A-88/07365**
**FR-A- 2 561 915**

- **DATABASE WPIL Week 9246, Derwent Publications Ltd., London, GB; AN 92-295490**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 556 110 B1

## Description

La présente invention a pour objet un vecteur pour au moins un principe actif thérapeutique ou cosmétique, à appliquer en surface par voie topique pour une action locale, sur la peau, les phanères, la conjonctive, la cornée, le conduit auditif ou les muqueuses nasales, buccales, gingivales, pharyngées ou vaginales, ce vecteur étant du type polymère particulaire, poreux, biocompatible et doué de propriétés d'adsorption réversible ou non dudit principe actif. Elle a également pour objet une composition thérapeutique ou cosmétique comprenant un tel vecteur.

On connaît déjà des compositions de ce type et notamment des collyres dans lesquels le vecteur est constitué par un polymère particulaire possédant des propriétés intrinsèques de bioadhésivité oculaire, favorisant sa rétention au niveau de la surface oculaire. Toutefois, la durée de rétention reste insuffisante en pratique ; il s'ensuit que le temps de contact du principe actif avec l'oeil reste limité, ce qui se traduit par une durée d'action et une biodisponibilité insuffisantes du principe actif et, partant, par une efficacité thérapeutique non satisfaisante.

La présente invention vise à remédier à cet inconvénient et, pour ce faire, elle propose un vecteur du type spécifié en introduction, qui se caractérise en ce qu'il comprend des molécules biocompatibles greffées à la surface dudit polymère particulaire, ces molécules présentant une affinité physique et/ou chimique pour la surface d'application et s'opposant à la migration dudit vecteur à travers cette surface, étant précisé que tout ou partie de ces molécules greffées peuvent en elles-mêmes être douées d'une activité thérapeutique ou cosmétique.

On comprendra que du fait de la présence de telles molécules sur le polymère particulaire, il en résultera une liaison réversible ou non du vecteur à la surface d'application, qui s'opposera à une élimination prématurée du vecteur sous l'effet des fluides biologiques ou, dans le cas d'un principe actif cosmétique, sous l'effet des agressions extérieures auxquelles ladite surface peut être soumise. Il s'ensuivra une prolongat ion du temps de rétention de ce vecteur sur ou au voisinage immédiat de la surface d'application et par conséquent une augmentation de la durée de contact du principe actif progressivement libéré par le polymère particulaire, avec ladite surface, ce qui contribuera à une meilleure biodisponibilité de ces principes actifs au niveau de cette surface.

On précisera que le greffage des molécules biocompatibles à la surface du polymère particulaire est un greffage par liaisons covalentes.

En outre, il entre également dans le cadre de la présente invention des vecteurs comprenant un polymère particulaire sur la surface duquel sort greffés à la fois des molécules présentant une affinité physique et/ou chimique pour la surface d'application et des molécules présentant une affinité physique et/ou chimique pour le principe actif. Dans ce cas, on observera une libération prolongée ou retardée des principes actifs, ce qui assure une diffusion régulière et prolongée de ces derniers au niveau de la surface d'application et, partant, contribue à l'obtention d'une biodisponibilité accrue desdits principes actifs. On assistera alors à un cumul de cet effet avec l'effet de prolongation du temps de rétention du vecteur sur ou au voisinage immédiat de la surface d'application et donc à une biodisponibilité encore accrue.

Le polymère particulaire entrant dans la constitution du vecteur selon l'invention peut être de toute nature pour autant qu'il possède les caractéristiques énumérées ci-avant et qu'il puisse être le siège d'un greffage de molécules biocompatibles. Il s'agira notamment de polymères ou copolymères comportant des fonctions OH, COOH, ester, $NH_2$, époxyde ou $CO-NH-CH_2-OH$. A ce titre, on citera par exemple les polymères de monomères tels que l'acide acrylique, l'acide méthacrylique, l'acide cyanoacrylique, l'acide cyanométhacrylique, un acrylate d'alkyle, un méthacrylate d'alkyle, un cyanoacrylate d'alkyle ou un cyanométhacrylate d'alkyle ; les copolymères de ces monomères avec un comonomère tel que le styrène ; et les polysaccharides tels que la cellulose et ses dérivés comme l'hydroxy cellulose et la carboxyméthyl-cellulose. Toutefois, préférence est donnée à la silice et aux polysiloxanes réticulés, qui présentent à leur surface des groupes silanolates (Si-OH) réactifs constituant des sites pour le greffage aptes à entrer en réaction avec de nombreuses molécules biocompatibles ; l'un des avantages de ce type de polymères réside dans leur parfaite inertie, tant sur le plan de la compatibilité avec les tissus vivants, qu'au niveau des mécanismes hydrolytiques qui peuvent avoir lieu au contact des fluides biologiques.

Par ailleurs, le polymère particulaire est de préférence sous la forme de nanoparticules dont la dimension est avantageusement comprise entre 10 et 500 nanomètres.

Selon un mode de réalisation de l'invention, les molécules greffées sont celles comportant un groupement ayant une affinité pour la surface d'application, et notamment une affinité protéique. Il peut notamment s'agir d'un groupement glycidyle, un groupement thiol (SH) libre ou protégé, un groupement isothiocyanate, un groupement aromatique ou un groupement disulfure activé.

A titre de molécules comportant un groupement glycidyle, on peut citer les composés du type (glycidoxyalkyl) trialkoxysilane tels que le (3-glycidoxypropyl)triméthoxysilane.

A titre de groupement SH protégé, on préfère un groupement du type sel de Bünte ($-S-SO_3M$ où M est un métal alcalin) ; des molécules du type sel de Bünte préférés sont constitués par les alkylthiosulfates de métal alcalin.

Les groupements susmentionnés présentent une affinité reconnue pour les protéines, celle-ci se traduisant généralement par la formation d'une liaison réversible avec les sites nucléophiles ($NH_2$, SH ou COOH) des protéines, avec pour résultat une rétention accrue du vecteur sur la surface d'application.

Ainsi, certains tissus contiennent des protéines riches en cystéine, un amino-acide porteur d'un groupe thiol qui leur confère la capacité de s'associer entre elles avec formation de ponts disulfure ; c'est le cas de la peau et des phanères qui contiennent des kératines (k-SH ou k-S-S-k).

Lorsque le vecteur selon l'invention comprenant un groupement thiol libre est amené en présence de ce type de tissus, on assistera en milieu neutre ou faiblement acide et en présence d'oxygène, conditions correspondant sensiblement aux conditions physiologiques, à une réaction entre les groupements thiol libres du vecteur et les groupes thiol libres de ces tissus ou les entités k-S-S-k susmentionnées, avec formation de ponts disulfure. Ce type de réaction est schématisé ci-après pour la kératine :

$$\text{Vecteur - SH + k - SH} \overset{\frac{1}{2}\,O_2}{\rightleftharpoons} \text{Vecteur - S - S - k} + H_2O$$

$$\text{Vecteur - SH + k - S - S - k} \rightleftharpoons \text{k - SH + Vecteur - S - S - k}$$

$$\text{Vecteur-S-S-k+Vecteur-SH} \rightleftharpoons \text{k-SH+Vecteur-S-S-Vecteur}$$

Il est à noter que toutes ces transformations sont en équilibre thermodynamique les unes avec les autres, mais que compte tenu de la nature polythiol du vecteur selon l'invention, le contrôle thermodynamique est en faveur de la formation de disulfures mixtes Vecteur - S - S - k.

De la même manière, les fonctions $-SSO_3M$ du vecteur de l'invention sont aptes à réagir, dans les conditions de pH ci-dessus, avec les groupes thiol libres des tissus ou avec les entités k-S-S-k ; pour la kératine, cette réaction peut se schématiser comme suit :

$$\text{Vecteur - }SSO_3M\text{ + k - SH} \rightleftharpoons \text{Vecteur - S - S - k + }MHSO_3$$

$$\text{k - S - S - k + }MHSO_3 \rightleftharpoons \text{k - SH + k - }SSO_3M$$

Cette dernière réaction est favorisée par des pH acides ou neutres. Si elle a lieu, elle ne peut que favoriser l'interaction vecteur - kératine dans la mesure où elle contribue à créer des entités k - SH plus réactives que les entités k - S - S - k.

La mise en oeuvre des molécules greffées à fonctions thiol libres ou protégées permet donc une interaction de nature chimique entre le vecteur et la surface sur laquelle ce dernier est appliqué, interaction assurant une prolongation du temps de rétention du vecteur, et donc des principes actifs, au voisinage immédiat de ladite surface.

La présente invention s'étend par ailleurs aux compositions thèrapeutiques ou cosmétiques destinées à être appliquées en surface par voie topique, comprenant un véhicule physiologiquement acceptable dans lequel est dispersé au moins un vecteur tel que décrit ci-dessus dont tout ou partie des molécules biocompatibles greffées possèdent en elles-mêmes une activité thérapeutique ou cosmétique et éventuellement au moins un principe actif à effet thérapeutique ou cosmétique adsorbé sur le polymère particulaire dudit vecteur.

Dans de telles compositions, lesdites molécules greffées possédant une activité thérapeutique ou cosmétique peuvent par exemple être celles habituellement utilisées en ophtalmologie pour suppléer à l'insuffisance de la sécrétion lacrymale ou restaurer le film lacrymal(éthers de cellulose, dérivés polyvinyliques ou polysaccharides) et dans ce cas lesdites compositions seront bienentendu à visée ophtalmologique.

La présente invention s'étend en outre aux compositions thérapeutiques ou cosmétiques destinées à être appliquées en surface par voie topique, comprenant un véhicule physiologiquement acceptable dans lequel est dispersé au moins un vecteur tel que décrit ci-dessus dont les molécules greffées n'ont pas d'activité thérapeutique ou cosmétique en elles-mêmes, et au moins un principe actif à effet thérapeutique ou cosmét.ique adsorbé sur le polymère particulaire dudit vecteur.

On peut envisager des principes actifs les plus divers, le vecteur selon l'invention ayant été conçu de manière à offrir un maximum de souplesse dans la mesure où il est possible de jouer sur la nature du polymère particulaire et des molécules greffées. La condition essentielle est que ces principes actifs puissent être adsorbés sur le polymère particulaire. A titre d'exemples de principes actifs on citera par exemple les silanols, les antioxydants (acide ascorbique et N-acétyl cystéine par exemple), les antibiotiques (pénicilline G, sulfate d'amikacine par exemple), les anti-inflammatoires etc...

La quantité de principe actif adsorbé sur le polymère particulaire varie largement suivant la nature du principe actif

et de son affinité vis-à-vis du polymère et éventuellement des molécules biocompatibles greffées sur ce dernier. Elle pourra par exemple constituer 0,05 à 20 % en poids du vecteur.

Quant au vecteur, il pourra habituellement représenter 0,1 à 40 % en poids de la composition.

Le véhicule physiologiquement acceptable est choisi pour assurer une dispersion stable du vecteur, ce qui signifie notamment qu'il ne doit pas être dissolvant de ce vecteur. Il pourra s'agir notamment de l'eau ou d'un liquide organique tel qu'un alcool, polyol comme le glycol ou la glycérine, un éther ou un ester de polyol, notamment un corps gras-, en particulier une huile végétale ou animale.

La proportion de ce véhicule ou sein de la composition pourra varier dans de larges limites pour autant qu'elle soit suffisante pour assurer la dispersion du vecteur. En pratique, cette proportion sera généralement de 20 à 99,6 % suivant la nature du véhicule.

Selon la nature du principe actif mis en oeuvre et des molécules greffées quand ces dernières présentent en elles-mêmes une activité physiologique ou cosmétique, les compositions trouvent leur application dans les domaines les plus divers (dermatologie, cosmétologie, ophtalmologie, oto-rhino-laryngologie, stomatologie et gynécologie).

La préparation du vecteur selon l'invention, bien que variable suivant la nature du polymère particulaire et des molécules biocompatibles greffées sur ce dernier, comporte généralement une phase de production du polymère particulaire, une phase de greffage sur ce polymère desdites molécules biocompatibles ou de précurseurs de ces dernières, suivie le cas échéant d'une phase de transformation de ces précurseurs en lesdites molécules biocompatibles.

Le principe de fabrication du polymère particulaire notamment sous forme de nanoparticules repose sur la mise en émulsion d'un monomère dans un milieu adéquat (par exemple eau dans le cas des monomères du type cyanoacrylate d'alkyle ou méthacrylate), suivie de la polymérisation, ce qui conduit à une suspension des nanoparticules dans ledit milieu. Cette technique étant bien connue de l'homme de métier, elle n'appelle pas de développements particuliers.

Dans le cas où le polymère particulaire est constitué par de la silice, par exemple sous forme nanoparticulaire, il peut être obtenu par hydrolyse d'un tétraalkoxysilane [$Si(OC_2H_5)_4$ par exemple] dans un mélange solvant eau-ammoniaque-alcool, ce qui libère l'acide silicique, suivie de la polymérisation de ce dernier dans ce même mélange solvant. Toutes informations utiles au sujet de cette synthèse bien connue sont disponibles dans J. Colloid Interface Sci. 81, 354 (1981) et J. Colloid Interface Sci. 26, 62 (1968).

On ajoutera que le polymère particulaire devra pour sa part présenter des sites de greffage. Ceux-ci pourront être présents sur le monomère de départ. Ils pourront également être générés en cours de la polymérisation du monomère ; c'est le cas de la silice ou des polysiloxanes qui présentent en surface de nombreux groupes silanolates (SiOH) réactifs qui jouent le rôle de sites de greffage.

Dans la phase suivante, les molécules biocompatibles ou des précurseurs de ces dernières sont amenées en réaction avec le polymère particulaire pour réaliser leur greffage sur la surface de ce dernier.

Ces molécules devront présenter une fonction libre apte à réagir avec tout ou partie des sites de greffage présents à la surface du polymère particulaire. si en plus de cette fonction libre lesdites molécules comportent par ailleurs un groupement fonctionnel susceptible de réagir avec les sites du polymère particulaire, alors soit la fonction libre desdites molécules est choisie pour présenter une réactivité avec les sites du polymère plus grande que celle du groupement fonctionnel, soit on opérera dans des conditions où ledit groupement fonctionnel ne participe pas à la réaction de greffage, soit ledit groupement fonctionnel est préalablement protégé puis déprotégé après la réaction de greffage.

A titre de fonction libre des molécules biocompatibles apte à entrer en réaction avec les sites de greffage du polymère particulaire, on peut citer les fonctions nucléophiles telles que OH, $NH_2$ ou SH, les fonctions amide, carboxyle, ester, ester activé ou chlorure d'acide ou encore la fonction silanolate (SiOH). Ainsi par exemple, la fonction OH est capable de se combiner avec des sites de greffage COOH (estérification catalysée par une base ou un acide) ou ester (transestérification avec catalyse acide ou basique) ;la fonction $NH_2$ est capable de se combiner avec des sites de greffage COOH après éventuelle activation de ces derniers par exemple par un carbodiimide ; la fonction COOH est capable de se combiner avec des sites de greffage OH (estérification catalysée par un acide ou une base) ; les fonctions SH, amide, carboxyle sont capables de se combiner avec des sites de greffage $CO\text{-}NH\text{-}CH_2\text{-}OH$ en milieu faiblement acide ; la fonction ester, ester activé ou chlorure d'acide est capable de réagir avec des sites de greffage $NH_2$ suivant une réaction de formation d'amide ; les fonctions nucléophiles en général sont capables de se combiner avec des sites de greffage époxyde habituellement en milieu pH voisin de 7 ; et la fonction silanolate est capable de se combiner avec des sites de greffage COOH, OH ou SiOH pour former respectivment des liaisons ester, Si-O-C ou Si-O-Si.

Comme indiqué ci-dessus, au lieu de mettre en oeuvre directement les molécules biocompatibles, il est possible également de mettre en oeuvre des précurseurs de ces dernières. Ces précurseurs sont choisis pour présenter deux fonctions distinctes de réactivité différente, seule l'une de ces fonctions étant capable de réagir avec les sites de greffage du polymère particulaire, l'autre étant capable de réagir avec une entité chimique pour conduire à la formation de la molécule biocompatible.

Ainsi par exemple, l'une de ces fonctions peut être constituée par une fonction -Si-OH et l'autre par un groupe glycidyle. Dans ce cas, le précurseur est d'abord mis à réagir, par sa fonction Si-OH, avec le polymère particulaire et, le greffage une fois effectué, le précurseur greffé est mis à réagir, par son groupe glycidyle, avec l'entité chimique pour réaliser la formation de la molécule biocompatible. Cette entité chimique peut par exemple être constituée par un thiosulfate de métal alcalin.

Dans le cas où le (3-glycidoxypropyl)triméthoxysilane est mis en oeuvre à titre de précurseur et un thiosulfate de sodium est mis en oeuvre à titre d'entité chimique, le schéma réactionnel est comme suit (P y symbolisant un polymère particulaire comportant des sites de greffage -Si-OH) ;

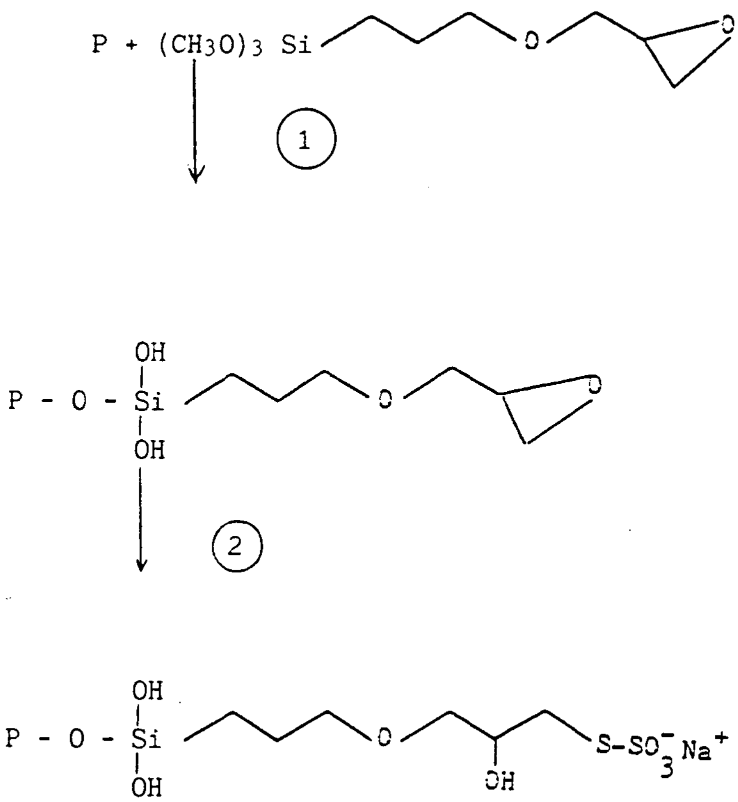


(1) : milieu aqueux alcoolique (eau - éthanol par exemple) acidifié par un acide tel que l'acide acétique ou une résine faiblement acide ; température modérée (40-50° C)

(2) : thiosulfate de sodium en milieu aqueux et maintien du pH à 6-8 par addition d'un acide tel que l'acide acétique.

L'invention est illustrée ci-après par un certain nombre d'exemples non limitatifs.

<u>Exemple 1</u> : Préparation de nanoparticules de silice greffées par des molécules de (3-glycidoxypropyl)triméthoxysilane

A un mélange de 800 ml d'éthanol, 20 ml d'eau distillée et 34 g d'ammoniaque à 25 %, on ajoute goutte à goutte, en environ 20 minutes, 60 g de tétraéthoxysilane. Le milieu est ensuite porté à 40-50° C environ, sous agitation. L'ammoniaque et une partie de l'éthanol sont ensuite évaporés jusqu'à un volume de 400 ml environ.

On fait suivre par une addition de 400 ml d'eau et une acidification à pH ~ 3,5-6 par exemple par une résine faiblement acide (type DOWEX CCR-2, marque déposée).

On ajoute alors 25 g de (3-glycidoxypropyl)triméthoxysilane et on agite 2 heures à 40-50° C.

On élimine ensuite la résine par filtration et les nanoparticules greffées ainsi obtenues sont conservées dans le mélange éthanol : eau (50:50).

Exemple 2 : Préparation de nanoparticules de silice greffées par un sel de Bünte

On soumet à une évaporation 500 ml du mélange éthanol : eau : nanoparticules greffées obtenu à l'exemple 1, pour éliminer l'éthanol.

Le produit restant et porté à 30° C et son pH est ajusté à environ 7 par une base telle que NaOH diluée.

On ajoute alors sous agitation, par fractions successives, 13,6 g de thiosulfate de sodium, en maintenant le pH à 6-8 par adjonction éventuelle d'acide acétique.

Lorsque le pH est stabilisé, on dialyse le mélange réactionnel afin d'éliminer les sels d'acide acétique et l'excès de thiosulfate.

Exemple 3 : Préparation d'un collyre pour suppléance lacrymale

A un mélange de 800 ml d'éthanol, 20 ml d'eau distillée et 34 g d'ammoniaque à 25 %, on ajoute goutte à goutte, en environ 20 minutes, 60 g de tétraéthoxysilane. Le milieu est ensuite porté à 40-50° C environ, sous agitation. L'ammoniaque et une partie de l'éthanol sont ensuite évaporés jusqu'à un volume de 400 ml environ.

On fait suivre par une addition de 400 ml d'eau et une acidification à pH ~ 3,5-6 par exemple par une résine faiblement acide (type DOWEX CCR-2, marque déposée).

Par ailleurs, dans 1 litre d'eau, on solubilise sous forte agitation, 1,5 g d'hyaluronate de sodium.

A la solution résultante, on ajoute successivement, éventuellement sous léger chauffage, 1, 2 g de p-hydroxybenzoate de méthyle sodé (agent conservateur), 0,3 g de p-hydroxybenzoate de propyle (agent conservateur) et 0,5 g du sel disodique de l'acide éthylénediamine tétraacétique.

On ajoute ensuite une quantité suffisante de la solution aqueuse éthanolique préalablement préparée, pour avoir 10 g de nanoparticules.

Le mélange obtenu est maintenu sous agitation pendant 15 minutes avant d'évaporer l'éthanol sous pression réduite.

La composition résultante est ensuite conditionnée en flacons stériles.

**Revendications**

1. Vecteur pour au moins un principe actif thérapeutique ou cosmétique à appliquer en surface par voie topique pour une action locale, sur la peau, les phanères, la conjonctive, la cornée, le conduit auditif ou les muqueuses nasales, buccales, gingivales, pharyngées ou vaginales, ce vecteur étant du type polymère particulaire, poreux, biocompatible et doué de propriétés d'adsorption réversibles ou non dudit principe actif et comportant des molécules biocompatibles greffées à sa surface, caractérisé en ce que lesdites molécules greffées sont des molécules biocompatibles comportant un groupement glycidyle, un groupement thiol (SH) libre ou protégé, un groupement isothiocyanate, un groupement aromatique ou un groupement disulfure activé, ces molécules présentant une affinité chimique pour la surface d'application et s'opposant à la migration dudit vecteur à travers cette surface.

2. Vecteur selon la revendication 1 caractérisé en ce que les molécules greffées sont choisies parmi les (glycidoxyalkyl) trialkoxysilanes et les molécules du type sels de Bünte.

3. Vecteur selon la revendication 1 ou 2, caractérisé en ce que le polymère particulaire est constitué par de la silice ou un polysiloxane réticulé.

4. Vecteur selon la revendication 1, 2 ou 3, caractérisé en ce que le polymère particulaire est constitué par des nanoparticules.

5. Vecteur selon l'une des revendications précédentes, caractérisé en ce que tout ou partie des molécules greffées sont douées d'une activité thérapeutique ou cosmétique.

6. Procédé de fabrication d'un vecteur selon l'une quelconque des revendications 1 à 5, caractérisé en ce qu'il comprend le greffage à la surface d'un polymère particulaire poreux, biocompatible et doué de propriétés d'adsorption réversible ou non de principe actif, de molécules biocompatibles comportant d'une part un groupement glycidyle, un groupement thiol (SH) libre ou protégé, un groupement isothiocyanate, un groupement aromatique ou un groupement disulfure activé, et d'autre part une fonction réactive, ledit polymère comportant pour sa part des sites de greffage aptes à entrer en réaction avec ladite fonction réactive des molécules biocompatibles.

7. Composition thérapeutique ou cosmétique destinée à être appliquée en surface par voie topique, caractérisée en ce qu'elle comprend un véhicule physiologiquement acceptable dans lequel est dispersé au moins un vecteur

selon l'une quelconque des revendications 1 à 5, dont tout ou partie des molécules biocompatibles greffées possèdent en elles-mêmes une activité thérapeutique ou cosmétique et éventuellement au moins un principe actif à effet thérapeutique ou cosmétique adsorbé sur le polymère particulaire dudit vecteur.

8. Composition thérapeutique ou cosmétique destinée à être appliquée en surface par voie topique, caractérisée en ce qu'elle comprend un véhicule physiologiquement acceptable dans lequel est dispersé au moins un vecteur selon l'une quelconque des revendications 1 à 5, dont les molécules greffées n'ont pas d'activité thérapeutique ou cosmétique en elles-mêmes, et au moins un principe actif à effet thérapeutique ou cosmétique adsorbé sur le polymère particulaire dudit vecteur.

9. Utilisation du vecteur selon l'une quelconque des revendications 1 à 5, pour la fabrication d'une composition thérapeutique ou cosmétique à appliquer en surface par voie topique pour une action locale, sur la peau, les phanères, la conjonctive, la cornée, le conduit auditif ou les muqueuses nasales, buccales, gingivales pharyngées ou vaginales.

## Claims

1. Vector for at least one therapeutic or cosmetic active principle for surface application by topical route in view of a local action, on the skin, the skin appendage, the conjunctiva, the cornea, the auditory canal or the nasal or buccal, gingival, pharyngeal or vaginal mucous, this vector being of a particulate, porous, biocompatible polymer type, having adsorption properties reversible or not of the said active principle and including biocompatible graft molecules at its surface, characterized by the fact that the said graft molecules are biocompatible molecules including a glycidyl group, a free or protected thiol (SH) group, an isothiocyanate group, an aromatic group or an activated disulfide group, these molecules having a chemical affinity for the application surface and counteracting the migration of the said vector through this surface.

2. Vector according to the claim 1, characterized by the fact that the graft molecules are chosen among the (glycidoxyalkyl) trialkoxysilane and the molecules of Bünte salt type.

3. Vector according to claim 1 or 2, characterized in that the particulate polymer is constituted of silica or of a cross-linked polysiloxane.

4. Vector according to claim 1,2 or 3, characterized in that the particulate polymer is constituted of nanoparticles.

5. Vector according to any of the preceding claims, characterized in that some or all of the graft molecules have a therapeutic or cosmetic activity.

6. Manufacturing process of a vector according to any of claims 1 to 5, characterized in that it comprises the grafting on to the surface of a particulate porous biocompatible polymer having adsorption properties reversible or not of the active principle, of biocompatible molecules including on one hand, a glycidyl group, a free or protected (SH) thiol group, a isothiocyanate group, an aromatic group or an activated disulfide group, and on the other hand, a reactive group, the said polymer including for its part grafting sites suitable for reacting with the said reactive group of the biocompatible molecules.

7. Therapeutic or cosmetic composition for surface application by topical route, characterized in that it comprises a physiogically acceptable vehicle in which is dispersed at least one vector according to any of claims 1 to 5, all or some of the biocompatible graft molecules of the vector having in themselves a therapeutic or cosmetic activity, and optionally has at least one active principle with therapeutic or cosmetic activity adsorbed on the particulate polymer of the said vector.

8. Therapeutic or cosmetic composition for surface application by topical route characterized in that it comprises a physiologically acceptable vehicle in which is dispersed at least one vector according to any of claims 1 to 5, said graft molecules not having any therapeutic or cosmetic activity in themselves, and has at least one active principle with therapeutic or cosmetic activity adsorbed on the particulate polymer of the said vector.

9. Use of a vector according to any of claims 1 to 5 for the manufacturing of a therapeutic or cosmetic composition for surface application by topical route in view of a local action, on the skin, the skin appendage, the conjunctiva, the cornea, the auditory canal, or the nasal or buccal, gingival, pharyngeal or vaginal mucous.

**Patentansprüche**

1. Ein Träger für mindestens einen therapeutischen oder kosmetischen Wirkstoff, der extern und örtlich zur lokalen Behandlung auf die Haut, die Anhangsgebilde der Haut, die Bindehaut, die Hornhaut, den Gehörgang oder die Nasen-, Mund-, Zahnfleisch-, Rachen- oder Vaginalschleimhäute aufgetragen wird, wobei dieser Träger vom partikelförmigen, porösen, biokompatiblen Typ ist, mit reversiblen Adsorptionseigenschaften dieses Wirkstoffs ausgestattet oder nicht, und auf seine Oberfläche gepfropfte biokompatible Moleküle hat, dadurch gekennzeichnet, daß diese aufgepfropften Moleküle biokompatible Moleküle sind, die eine Glycidylgruppe, eine freie oder geschützte Thioalkoholgruppe (SH), eine Isothiocyanatgruppe, eine aromatische Gruppe oder eine aktivierte Bisulfidgruppe enthalten, und daß diese Moleküle eine chemische Affinität zur Auftragsfläche aufweisen und der Migration dieses Trägers durch diese Fläche entgegenwirken.

2. Ein Träger nach Anspruch 1, dadurch gekennzeichnet, daß die aufgepfropften Moleküle unter den (Glycidoxyalkyl)Trialkoxysilanen und den Molekülen vom Büntesalztyp gewählt werden.

3. Ein Träger nach Anspruch 1 oder 2, dadurch gekennzeichnet, daß das Partikelpolymer aus Siliziumdioxyd oder einem vernetzten Polysiloxan besteht.

4. Ein Träger nach Anspruch 1, 2 oder 3, dadurch gekennzeichnet, daß das Partikelpolymer aus Nanopartikeln besteht.

5. Ein Träger nach einem der obigen Ansprüche, dadurch gekennzeichnet, daß alle oder ein Teil der aufgepfropften Moleküle eine therapeutische oder kosmetische Wirkung haben.

6. Ein Fertigungsverfahren für einen Träger nach einem der Ansprüche 1 bis 5, dadurch gekennzeichnet, daß es das Aufpfropfen von biokompatiblen Molekülen, die einerseits eine Glycidylgruppe, eine freie oder geschützte Thioalkoholgruppe (SH), eine Isothiocyanatgruppe, eine aromatische Gruppe oder eine aktivierte Bisulfidgruppe und andrerseits eine reaktive Funktion enthalten, auf die Oberfläche eines partikelförmigen, porösen, biokompatiblen Trägers umfaßt, welcher mit reversiblen Adsorptionseigenschaften des Wirkstoffs ausgestattet ist oder nicht, wobei dieses Polymer seinerseits mit Pfropfstellen versehen ist, die mit der obigen reaktiven Funktion des biokompatiblen Moleküls reagieren können.

7. Eine therapeutische oder kosmetische Zusammensetzung zur örtlichen und externen Anwendung, dadurch gekennzeichnet, daß sie ein physiologisch annehmbares Vehikel enthält, in dem mindestens ein Träger nach einem der Ansprüche 1 bis 5 dispergiert ist, dessen aufgepfropfte biokompatible Moleküle alle oder teilweise selbst eine therapeutische oder kosmetische Wirkung haben, und eventuell mindestens einen therapeutischen oder kosmetischen Wirkstoff, der auf dem Partikelpolymer dieses Trägers adsorbiert ist.

8. Eine therapeutische oder kosmetische Zusammensetzung zur örtlichen und externen Anwendung, dadurch gekennzeichnet, daß sie ein physiologisch annehmbares Vehikel enthält, in dem mindestens ein Träger nach einem der Ansprüche 1 bis 5 dispergiert ist, dessen aufgepfropfte biokompatible Moleküle keine therapeutische oder kosmetische Wirkung haben, und mindestens einen therapeutischen oder kosmetischen Wirkstoff, der auf dem Partikelpolymer dieses Trägers adsorbiert ist.

9. Verwendung des Trägers nach einem der Ansprüche 1 bis 5 zur Herstellung einer therapeutischen oder kosmetischen Zusammensetzung zur örtlichen und externen Anwendung auf der Haut, den Anhangsgebilden der Haut, der Bindehaut, der Hornhaut, dem Gehörgang oder den Nasen-, Mund-, Zahnßeisch-, Rachen- oder Vaginalschleimhäuten.